(19) Europäisches Patentamt
European Patent Office
Office européen des brevets

(11) Veröffentlichungsnummer: **0 303 184**
**A2**

(12) **EUROPÄISCHE PATENTANMELDUNG**

(21) Anmeldenummer: **88112700.5**

(22) Anmeldetag: **04.08.88**

(51) Int. Cl.⁴: **C07C 43/178 , C07C 43/14 , C07C 69/145 , C07D 309/12**

Die Bezeichnung der Erfindung wurde geändert (Richtlinien für die Prüfung im EPA, A-III, 7.3).

(30) Priorität: **08.08.87 DE 3726511**

(43) Veröffentlichungstag der Anmeldung: **15.02.89 Patentblatt 89/07**

(84) Benannte Vertragsstaaten: **CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Becker, Rainer, Dr.**
**22 Im Haseneck**
**D-6702 Bad Duerkheim(DE)**
Erfinder: **Seufert, Walter Dr.**
**Laerchenweg 19**
**D-6720 Speyer(DE)**
Erfinder: **Buschmann, Ernst,Dr.**
**Georg-Ludwig-Krebs-Strasse 10**
**D-6700 Ludwigshafen(DE)**
Erfinder: **Brueckner, Christiane, Dr.**
**Bad-Aussee-Strasse 55**
**D-6700 Ludwigshafen(DE)**

# (54) 1,1-Dialkoxy- oder 1,1-(Alpha,Omega-Alkylendioxy)-non-2-yn-9-ol und deren OH-geschützte Derivate.

(57) 1,1-Dialkoxy- oder 1,1-(α,ω-Methylendioxy)-non-2-in-9-ol und deren OH-geschützte Derivate der allgemeinen Formel I,

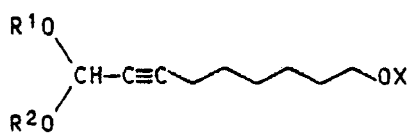

in der die Substituenten
$R^1$, $R^2$ $C_1$-$C_6$-Alkyl oder gemeinsam eine Alkylenkette mit 2 bis 5 Kohlenstoffatomen und
X Wasserstoff oder eine abspaltbare Schutzgruppe
bedeuten,

und deren Herstellung und Verwendung als Zwischenprodukte.

EP 0 303 184 A2

## 1,1-Dialkoxy- oder 1,1-(α,ω-Methylendioxy)-non-2-in-9-ol und deren OH-geschützte Derivate

Es wurde gefunden, daß 1,1-Dialkoxy- oder 1,1-(α,ω-Methylendioxy)-non-2-in-9-ol und deren OH-geschützte Derivate der allgemeinen Formel I

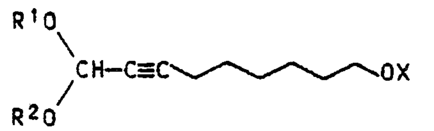 (I),

in der die Substituenten
$R^1$, $R^2$ $C_1$-$C_6$-Alkyl oder gemeinsam eine Alkylenkette mit 2 bis 5 Kohlenstoffatomen und
X Wasserstoff oder eine abspaltbare Schutzgruppe
bedeuten,

als Zwischenprodukte zur Synthese von E-7, Z-9-Dodecadienylacetat, dem Pheromon des bekreuzten Traubenwicklers Lobesia botrana geeignet sind. Dieser Wirkstoff wurde 1973 erstmals beschrieben [Mitteilungen der schweizerischen entomologischen Gesellschaft 46, 71-73 (1973), US-A-3 845 108, DE-A-24 40 759]. Das dort ebenfalls beschriebene Herstellverfahren weist 10 meist aufwendige Verfahrensschritte auf und ist somit sehr umständlich; zur Synthese großer Mengen, wie sie zur großflächigen Verwendung von Pheromonwirkstoffen zur Insektenkontrolle mittels der Konfusionsmethode erforderlich sind, ist es nicht geeignet.

Wegen der Vorteile der biologischen Schädlingsbekämpfung mit Pheromonen (sehr spezifische, nützlingsschonende Wirkstoffe, keine Resistenzerscheinungen, gute biologische Abbaubarkeit, außerordentliche geringe Toxizität) ist eine in technischem Maßstab wirtschaftliche Synthese dringend gesucht.

Der vorliegenden Erfindung liegt damit die Aufgabe zugrunde, ein technisch einfach durchführbares Herstellverfahren für den Pheromonwirkstoff zur Verfügung zu stellen. Diese Aufgabe wurde durch Bereitstellung des neuen 1,1-Dialkoxy-oder 1,1-(α,ω-Methylendioxy)-non-2-in-9-ols und deren OH-geschützten Derivate

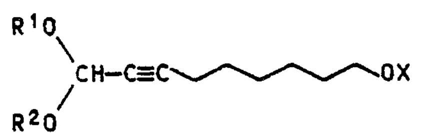 (I)

als Zwischenprodukte gelöst, die gut zugänglich sind und nach Hydrierung und Verseifung die bekannten substituierten Nonenale (III) liefern

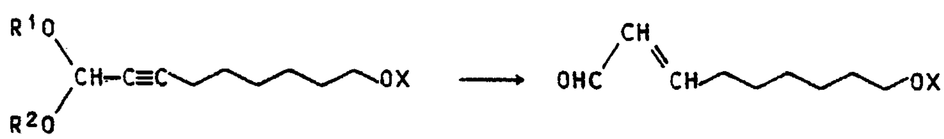

I III

Die Verwendung der Verbindungen der Nonenale (III) zur Synthese des gesuchten Pheromonwirkstoffes ist beschrieben (GB-A-2 098 609 bzw. Liebigs Ann. Chem. (1981) 1705).

In den eingangs definierten Noninolderivaten I stehen die Reste $R^1$ und $R^2$ bevorzugt für eine verzweigte oder unverzweigte niedermolekulare Alkylgruppe z.B. für Methyl, Ethyl, Propyl, iso-Propyl, Butyl, iso-Butyl, sec.-Butyl, Pentyl oder Hexyl. Besonders bevorzugt sind Ethyl- oder Methylreste, da diese Verbindungen besonders leicht zugänglich sind. $R^1$ und $R^2$ können auch miteinander unter Ausbildung eines 5- bis 8-gliedrigen Ringes verbunden sein. In diesem Fall kommen insbesondere 5- oder 6-gliedrige Ringe, z.B. das 1,3-Dioxan- oder 1,3-Dioxolansystem in Betracht.

Die erfindungsgemäßen Derivate des Non-2-in-9-ols I können hergestellt werden durch Umsetzung der bekannten Acetylenderivate des Typs II

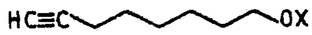 (II)

[J. Chem. Soc. (Chem. Comm.) 874 (1973)] mit Orthoameisensäureester in Gegenwart oder nach Einwirkung einer starken Base wie beispielsweise Alkalimetallamid oder einer Grignard-Verbindung. Als Alkalimetallamide seien beispielsweise Lithium- oder Kaliumamid ($LiNH_2$, $NaNH_2$) oder substituierte Amide wie Lithiumdiisopropylamid genannt. Grignard-Verbindungen sind Alkylmagnesiumchloride, -bromide oder -iodide wie Methylmagnesiumbromid, Ethylmagnesiumchlorid oder Methylmagnesiumiodid.

Die Basenmenge ist nicht besonders kritisch, im allgemeinen verwendet man stöchiometrische Mengen oder einen geringen Überschuß, z.B. 10 bis 20 Mol% Überschuß, bezogen auf das Acetylenderivat I. Höhere Überschüsse sind möglich, in der Regel aber nicht nötig.

Die Umsetzung wird bei einer Temperatur zwischen 50 und 150 °C bei Normaldruck oder gegebenenfalls unter Eigen- oder Überdruck von 1,01 bis 10 bar, in einem geeigneten aprotischen Lösungsmittel, wie Diethylether, Methyl-tert.-butylether, Tetrahydrofuran, Dioxan oder Glykolethern vorgenommen. Als Orthoester wird vorteilhaft handelsüblicher Orthoameisensäuretrimethylester oder Orthoameisensäuretriethylester verwendet. Die unsymmetrisch substituierten und cyclischen Acetale I lassen sich durch Verwendung der entsprechenden, an sich bekannten oder nach allgemein bekannten Methoden herstellbaren Orthoameisensäureestern gewinnen oder können durch die allgemein bekannte Methode der Umacetalisierung aus symmetrisch acyclischen substituierten Acetalen I generiert werden. Bezogen auf das Acetylenderivat I werden im allgemeinen äquimolare Mengen oder ein geringer Über- oder Unterschuß des Orthoesters verwendet.

Als abspaltbare Schutzgruppen X eignen sich basenstabile Hydroxyschutzgruppen, z.B. $C_4$-$C_{20}$-tert.-Alkylgruppen, bevorzugt $C_4$-$C_{20}$-tert.-Alkylgruppen, die in 1-Position ein tertiäres Kohlenstoffatom tragen, wie tert.-Butyl, 1,1-Dimethylprop-1-yl, 1,1-Dimethylbut-1-yl, 1,1,2-Trimethylprop-1-yl, 1,1-Dimethylpent-1-yl, 1,1,2-Trimethylbut-1-yl, 1,1,3-Trimethyl-but-1-yl, 1-Ethyl-1-methyl-but-1-yl, 1,1-Dimethylhex-1-yl und 1,1-Dimethyl-2-ethylbut-1-yl; $C_3$-$C_{20}$-Trialkylsilylgruppen, bevorzugt $C_3$-$C_8$-Trialkylsilylgruppen, wie Trimethylsilyl, Triethylsilyl, Tri-n-propylsilyl, Tri-isopropylsilyl, Tri-n-butylsilyl, Dimethylethylsilyl, Diethylmethylsilyl, Dimethyl-n-propylsilyl, Dimethyl-iso-propylsilyl, Dimethyl-n-butylsilyl und Dimethyl-tert.-butylsilyl; Benzyl; Acyle, z.B. Alkanoyle, wie Acetyl, Propionyl und Butyryl; Benzoyl; acyclische acetalische Gruppen, z.B. $C_2$-$C_{20}$-Alkoxymethoxy, bevorzugt $C_2$-$C_9$-Alkoxymethoxy, wie Methoxymethoxy, Ethoxymethoxy, n-Propoxymethoxy, iso-Propoxymethoxy, n-Butoxymethoxy, iso-Butoxymethoxy, sec.-Butoxymethoxy, tert.-Butoxymethoxy, n-Hexoxymethoxy und n-Octoxymethoxy; $C_3$-$C_{20}$-1-Alkoxy-ethoxy, bevorzugt $C_3$-$C_{10}$-1-Alkoxy-ethoxy, wie 1-Methoxy-ethoxy, 1-Ethoxy-ethoxy, 1-n-Propoxy-ethoxy, 1-iso-Propoxy-ethoxy, 1-n-Butoxy-ethoxy, 1-iso-Butoxy-ethoxy, 1-sec.-Butoxy-ethoxy, 1-tert.-Butoxy-ethoxy, 1-n-Hexoxy-ethoxy und 1-n-Octoxyethoxy; cyclische acetalische Gruppen, wie 2-Furanyl, 2-Tetrahydrofuranyl, 2-Pyranyl, 2-Tetrahydropyranyl, 1,3-Dioxan-2-yl, 1,3-Dioxan-4-yl und 1,4-Dioxan-2-yl.

Die Abspaltung der Schutzgruppe zur Herstellung der Verbindungen I mit der Bedeutung von X = Wasserstoff kann in an sich bekannter Weise, z.B. durch saure Hydrolyse erfolgen.

## Herstellungsbeispiele

### Beispiel 1

a) Zu einer wie üblich bereiteten Grignard-Lösung aus 51,1 g (2,1 mol) Magnesium und 228 g (2,1 Mol) Ethylbromid in 300 ml THF wird 364 g (2,0 Mol) 8-tert.-Butoxyoctin gegeben und nach 1 Stunde Rückfluß (wobei die Temperatur von 60 auf 75 °C steigt) und gutem Rühren mit 296 g (2 Mol) Triethylorthoformiat versetzt und am Rückfluß gehalten, bis die Umsetzung gaschromatographisch bestimmt vollständig ist (ca. 15 Std.) Anschließend wird in Eiswasser gegeben, mit Ammoniumchlorid gelöst und mit Methyl-tert.-butylether 3 mal extrahiert. Nach Auswaschen und Einengen verbleiben 555 g eines hellbraunen Öls, welches nach gaschromatographischer Untersuchung einen Gehalt von 92 % 1,1-Diethoxy-9-tert.-butoxy-non-2-in (entsprechend 91 % Ausbeute) aufweist.

b) 940 g 1,1-Diethoxy-9-hydroxy-non-2-in (roh, 71 %ig) werden mit 390 g Pyridin vorgelegt und bei 60 bis 70°C mit 502 g Acetanhydrid versetzt. Nach 4-stündigem Rühren bei ~ 70°C wird auf Eiswasser gegossen, auf pH = 6 gestellt und mit Methyl-tert.-butylether 3 mal extrahiert, mit Wasser gewaschen und eingeengt. Erhalten werden 1 160 g 1,1-Diethoxy-9-hydroxy-non-2-in (Ausbeute nach Destillation $Kp_{0,4}$ = 135-137°C: 650 g (97 %ig), d.h. 85 % Ausbeute).

Beispiel 2

Das im vorstehenden Beispiel eingesetzte Hydroxynoninderivat kann außer durch Umsetzung des Oktinols mit Orthoester auch aus dem THP-Derivat durch Abspaltung der THP-Schutzgruppe erhalten werden; diese Möglichkeit ist insofern überraschend, als im eingesetzten Diethoxynonin-THP-ether zwei acetalische Funktionen vorliegen, von denen selektiv nur die THP-Funktion gespalten wird, während die Diethoxyfunktion erhalten bleibt:

1 230 g 1,1-Diethoxy-9-tetrahydropyranyloxynon-2-in werden in 5 l Ethanol mit 25 g p-Toluolsulfonsäure über 20 Std. bei 20°C gerührt, dann mit Natriumhydrogencarbonat/$H_2O$ neutralisiert, in 5 l $H_2O$ eingerührt und mit Methyl-tert.-butylether extrahiert. Nach Auswaschen mit Wasser und Einengen verbleiben 940 g Rohprodukt (1,1-Diethoxy-9-hydroxynon-2-in). Der gaschromatographisch bestimmte Gehalt liegt bei 70 %, eine destillative Reinigung ist wegen der thermischen Instabilität nicht möglich, jedoch kann das Rohprodukt direkt für die Stufe b) des vorstehenden Beispiels 1 verwendet werden.

Nach den vorstehenden Angaben werden mit entsprechender Abwandlung die nachstehenden Verbindungen hergestellt:

| $R^1$ | $R^2$ | X | Kp [°C/mm Hg] | 270 MHz-$^1$-NMR δ [ppm] ($CDCl_3$) |
|---|---|---|---|---|
| $C_2H_5$ | $C_2H_5$ | 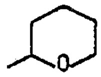 | 165-168 / 0,4 | |
| $C_2H_5$ | $C_2H_5$ | tert.-Butyl | 134-136 / 0,7 | |
| $C_2H_5$ | $C_2H_5$ | H | | 1,1-1,6 (m,14H), 2,23 (t,2H), 3,6 (m,6H), 5,22 (s,1H) |
| $C_2H_5$ | $C_2H_5$ | $COCH_3$ | 135-137 / 0,4 | |
| $C_2H_5$ | $C_2H_5$ | 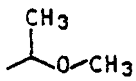 | | |
| $C_2H_5$ | $C_2H_5$ | 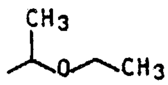 | | |
| $C_2H_5$ | $C_2H_5$ | 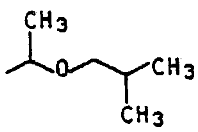 | | |
| $C_2H_5$ | $C_2H_5$ | $Si(CH_3)_3$ | | |
| $CH_3$ | $CH_3$ | 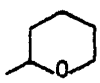 | | |
| $CH_3$ | $CH_3$ | tert.-Butyl | | |
| $CH_3$ | $CH_3$ | H | | |
| $CH_3$ | $CH_3$ | $COCH_3$ | | |
| $CH_3$ | $CH_3$ | 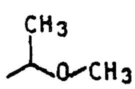 | | |
| $CH_3$ | $CH_3$ | 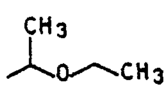 | | |
| $CH_3$ | $CH_3$ | 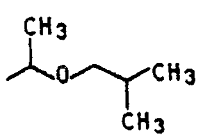 | | |
| $CH_3$ | $CH_3$ | $Si(CH_3)_3$ | | |

## Ansprüche

1. 1,1-Dialkoxy- oder 1,1-(α,ω-Methylendioxy)-non-2-in-9-ol und deren OH-geschützte Derivate der allgemeinen Formel I

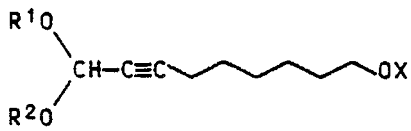 (I),

in der die Substituenten
$R^1$, $R^2$ $C_1$-$C_6$-Alkyl oder gemeinsam eine Alkylenkette mit 2 bis 5 Kohlenstoffatomen und
X Wasserstoff oder eine abspaltbare Schutzgruppe
bedeuten.

2. Verfahren zur Herstellung der Verbindungen der Formel I gemäß Anspruch 1, dadurch gekennzeichnet, daß man ein entsprechendes Acetylenderivat der Formel

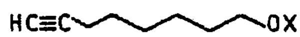 (II)

worin X für eine Alkoholschutzgruppe steht, in Gegenwart bzw. nach Einwirkung einer Base mit einem Orthoameisensäureester umsetzt und gegebenenfalls die Schutzgruppe in an sich bekannter Weise abspaltet.

3. Verwendung eines Derivates des Non-2-in-9-ols der Formel I als Zwischenprodukt zur Synthese von E-7, Z-9-Dodecadienylacetat.